# EUROPEAN PATENT APPLICATION

(11) **EP 3 960 129 A1**
(43) Date of publication of application: **02.03.2022**
(21) Application number: 21785740.8
(22) Date of filing: 18.01.2021
(51) Int. Cl.: A61F 2/24

(54) **PROSTHETIC HEART VALVE**

(30) Priority: 15.07.2020 CN 202010680139; 15.07.2020 CN 202021389484 U
(71) Applicant: Shanghai Trulive Medtech Co., Ltd, Shanghai 201206 (CN)
(72) Inventor: ZHAO, Jing, Shanghai 201108 (CN)
(74) Representative: Argyma
(86) International application number: PCT/CN2021/072482
(87) International publication number: WO 2022/012011

(57) **Abstract**

A prosthetic heart valve includes a stent body for supporting prosthetic valve leaflets and an anchoring frame for anchoring the stent body to annulus. The anchoring frame is configured at a pre-set position located close to an outflow tract, the stent body is configured on an outer side of the anchoring frame and at a pre-set position located far away from the outflow tract, and the anchoring frame abuts against and is connected to the stent body.

## Description

### BACKGROUND OF THE INVENTION

### Field of the Invention

The present invention relates to the technical field of medical instruments, and more specifically, to a heart valve prosthesis for implanting into a heart.

### Description of the Prior Art

The heart contains four heart cavities; the left atrium and left ventricle are located on the left side of the heart, and the right atrium and right ventricle are located on the right side of the heart. A ventricle inflow tract is formed between the atrium and the ventricle, the left ventricle and the aorta form the left ventricle outflow tract, and the right ventricle and the pulmonary artery form the right ventricle outflow tract. There is a valve with functions of a "one-way valve" at the ventricle inflow tract and the ventricle outflow tract to ensure the normal blood flow in the heart cavity. When there is a problem with the valve, the hemodynamics of the heart change and the heart function is abnormal, resulting in a condition called a valvular heart disease.

The mitral regurgitation may cause reconstruction of cardiac muscles, lead the ventricle to dilate progressively, and finally cause heart failure. The transcatheter mitral valve replacement (TMVR) adopts a method of intervening a catheter, wherein the prosthetic valve is compressed to a delivery system in vitro and transported to the human mitral valve annulus, and then the prosthetic valve is released and anchored at the mitral valve annulus to replace the native valve. Compared with surgery, TMVR does not require extracorporeal circulation devices, has a small trauma, a quick recovery for the patients, and can significantly improve the hemodynamic index of the patients after operation.

Although the transcatheter mitral valve replacement technology develops rapidly, there are still some recognized challenges in the design of valves, for example:
1. Compared with the aortic valve, the native valve annulus of the mitral valve have a larger diameter, and correspondingly the valve leaflet of the prosthetic valve has a larger area. The greater the area of the valve leaflet is, the poorer the anti-fatigue performance is.
2. For the existing mitral valve prosthesis, the delivery catheter has a greater diameter, thereby increasing the difficulty in transporting and the risk in damaging the blood vessels.
3. The atrioventricular valve composite has a complex structure, wherein if the subvalvular height of the prosthesis valve is too large, the structure and functions of the primary heart may be affected, heart tissues such as the papillary muscle may be touched to cause abnormality while being prone to lead blockade of the left ventricle outflow tract (LVOT), and adverse postoperative effects will be induced.

The tricuspid valve also has the problems of too large subvalvular height of the valve and blockade of the right ventricle outflow tract.

### SUMMARY OF THE INVENTION

The present invention provides a prosthetic heart valve, which may solve the above drawbacks in the prior art.

The technical solution of the present invention is as follows:
A prosthetic heart valve includes a stent body for supporting prosthetic valve leaflets and an anchoring frame for anchoring the stent body to annulus, wherein the anchoring frame is configured at a pre-set position located close to an outflow tract, the stent body is configured outside the anchoring frame and at a pre-set position located far away from the outflow tract, and the anchoring frame abuts against and is connected to the stent body.

Implanting the heart valve at the annulus is used to replace the native valve leaflets to realize functions of opening and closing a blood channel. Compared with the existing single-layered stent, the stent body and the anchoring frame are used for supporting the valve leaflets and anchoring respectively, and a radial size of the stent body is smaller so that a size of the prosthetic valve leaflet placed on the stent body is also smaller, thereby improving the fatigue resistance of the valve leaflet. The size of the valve leaflet is smaller, so the amount of a material of the valve leaflets is reduced accordingly. Compared with a double-layered stent with an internal-external nested structure, the stent body and the anchoring frame of the present invention are of a left-right parallel connected structure, and a size of the anchoring frame is relatively smaller, so the amount of a material of the anchoring frame and a material of a skirt covered on the anchoring frame are fewer. The fewer the amount of the material is, the smaller the size is after pressing and holding, and the easier the stent is pressed, held and loaded.

Studies have shown that if the prosthetic mitral valve may drive the blood to flow along a side wall of the ventricle, the blood may turn smoothly to create a large vortex, so as to eject the blood to the aorta and further to flow through the whole body. For the heart valve with the left-right structure, as the valve leaflets are located on the stent body and the stent body is biased more towards one side of the side wall of the ventricle, the blood flow may tend to flow along the side wall of the ventricle, which is more beneficial to maintain a natural "blood vortex " for the left atrium, thereby promoting the recovery for the ventricle functions, especially for the vulnerable patients with severely-damaged heart conditions. Preferably, a peripheral side of the anchoring frame and a peripheral side of the stent body abut against each other and are connected with each other at a position where the two abut against each other. With this structure, a surface-to-surface contact may be formed between the anchoring frame and the stent body, so the connection between the two is more firm.

Preferably, the position where the anchoring frame abuts against the stent body is of a single-layered structure, which may save the amount of the material for the heart valve.

Preferably, the anchoring frame or the stent body is of a non-enclosed structure. The non-enclosed structure may reduce the amount of the material for the anchoring frame or the stent body.

Preferably, the anchoring frame is of an opening structure along an axial direction, the anchoring frame abuts against a periphery of the stent body at the opening, and an opening end of the anchoring frame is connected to the stent body; or the stent body is of an opening structure along an axial direction, the stent body abuts against the periphery of the anchoring frame at the opening, and an opening end of the stent body is connected to the anchoring frame. The opening structure is disposed along the axial direction such that the amount of the materials for the valve stent and for the skirt covered on the valve stent is further reduced without affecting the functions of the anchoring frame or the stent body, thereby further facilitating pressing, holding and deploying the valve prosthesis.

Preferably, at the position where the anchoring frame abuts against the stent body, a part of the anchoring frame is of an opening structure, a part of the stent body abutting against the part of the anchoring frame is of a non-opening structure, other parts of the anchoring frame are of non-opening structures, and a part of the stent body abutting against the other part of the anchoring frame is of the opening structure.

Preferably, the anchoring frame has a notch structure, the notch structure is located at an outflow segment of the anchoring frame, and the notch structure is configured to face towards the outflow tract. The notch structure prevents the anchoring frame from crossing the ventricle wall surface, so the prosthetic heart valve may not cause the ventricle wall to abut more close to a right side of the aortic valve or a left side of the pulmonary valve. At the same time, the notch structure configured at the outflow segment reduces the subvalvular height of the frame portion, and reduces the risk of outflow tract obstruction.

Preferably, the valve includes prosthetic valve leaflets, and the valve leaflets are arranged at the main body portion. One end of the valve leaflet is connected to the main body portion, and free ends of the valve leaflet are engaged with each other.

When the valve is in a working state, the native valve leaflets are replaced by the prosthetic valve leaflets to realize functions of opening and closing a blood channel.

In order to prevent a relative movement of the heart valve in a delivery system and make the heart valve be smoothly implanted to a pre-set location during the transporting process while realizing deploying and removing, the heart valve further includes at least one connecting tab, and the heart valve is connected to the delivery system by the connecting tab; the connecting tab is disposed at an end of the inflow segment or an end of the outflow segment of the stent body, or the connecting tab is disposed at an end of the inflow segment or an end of the outflow segment of the anchoring frame.

Preferably, the heart valve further includes a skirt for sealing. The skirt is used for realizing sealing to ensure that a single channel of the blood is an outflow tract end flowing from an inflow tract end of the heart valve to the valve prosthesis, which may effectively prevent the peripheral leakage and the regurgitation of the valve.

Compared with the conventional art, the present invention has the following beneficial effects:
First, compared with the existing single-layered structure, the stent body and the anchoring frame of the present invention are used for supporting the valve leaflet and anchoring respectively, and a radial size of the stent body is smaller so that a size of the prosthetic valve leaflet is also smaller, thereby improving the fatigue resistance of the valve leaflet; the size of the valve leaflet is smaller, so the amount of a material of the valve leaflet is reduced; compared with a double-layered stent with an internal-external nested structure, the stent body and the anchoring frame of the present invention are of a left-right parallel connected structure, and a size of the anchoring frame is reduced, so the amount of a material of the anchoring frame and a material of the skirt are fewer; the fewer the amount of the material is, the smaller the size is after pressing and holding, and the easier the stent is pressed, held and loaded. Moreover, for the heart valve with the left-right structure, as the valve leaflet is located on the stent body and the stent body is biased more towards one side of the side wall of the ventricle, the blood flow may tend to flow along the side wall of the ventricle, which is more beneficial to maintain a natural " blood vortex " for the left atrium, thereby promoting the recovery for the ventricle functions, especially for the vulnerable patients with severely-damaged heart conditions.

Second, the stent body and the anchoring frame of the present invention are of the left-right parallel connected structure so that a surface-to-surface contact is formed between the anchoring frame and the stent body, so the connection between the two are more firm.

Third, the anchoring frame of the present invention is further configured with the notch structure, wherein when the notch structure faces towards the outflow tract, the notch structure prevents the anchoring frame from crossing the ventricle wall surface, so the prosthetic heart valve may not cause the ventricle wall to abut more close to a right side of the aortic valve or a left side of the pulmonary valve, thereby reducing the effects of the prosthetic heart valve on the functions of the heart; when the anchoring frame is of a non-enclosed structure along the axial direction, the opening structure is disposed along the axial direction such that the amount of the materials for the valve stent and for the skirt covered on the valve stent is further reduced without affecting the functions of the anchoring frame or the stent body, thereby further facilitating pressing, holding and releasing the valve prosthesis.

Certainly, any one product for implementing the present invention is unnecessary to achieve all the above advantages at the same time.

### BRIEF DESCRIPTION OF THE DRAWINGS

Fig. 1 is a partial structural schematic diagram of a prosthetic heart valve according to Embodiment 1 of the present invention;
Fig. 2 is a top diagram of the prosthetic heart valve according to Embodiment 1 of the present invention;
Fig. 3 is an illustrative perspective diagram of the prosthetic heart valve according to Embodiment 1 of the present invention;
Fig. 4 is a structural schematic diagram of an anchoring frame according to Embodiment 1 of the present invention;
Fig. 5 is a top structural schematic diagram of the anchoring frame according to Embodiment 1 of the present invention;
Fig. 6 is another illustrative perspective diagram of the prosthetic heart valve according to Embodiment 1 of the present invention;
Fig. 7 is another structural schematic diagram of the anchoring frame according to Embodiment 1 of the present invention;
Fig. 8 is a structural schematic diagram of a stent body according to Embodiment 1 of the present invention;
Fig. 9 is a top diagram of the stent body according to Embodiment 1 of the present invention;
Fig. 10 is a structural schematic diagram of another stent body according to Embodiment 1 of the present invention;
Fig. 11 is a top diagram of another stent body according to Embodiment 1 of the present invention;
Fig. 12 is a schematic diagram of implanting the prosthetic heart valve into the heart according to Embodiment 1 of the present invention;
Fig. 13 is a diagram of the prosthetic heart valve according to Embodiment 3 of the present invention;
Fig. 14 is a diagram of another prosthetic heart valve according to Embodiment 3 of the present invention;
Fig. 15 is a diagram of another prosthetic heart valve according to Embodiment 3 of the present invention;
Fig. 16 is an illustrative perspective diagram of the prosthetic heart valve according to Embodiment 4 of the present invention.

References for numerals: stent body-110; main body inflow segment-111; main body transition segment-112; main body outflow segment-113; hanging tab-114; anchoring frame-210; inflow segment-211; transition segment-212; outflow segment-213; fixing tab-214; abutting position-215; notch structure-216; frame opening end-300; main body opening end-301.

### DESCRIPTION OF THE PREFERRED EMBODIMENTS

In order to describe the structural characteristics of the present invention more clearly, the terms "proximal end" and "distal end" are used as orientation words, wherein "proximal end" means an end close to the tip of the heart during surgery, and "distal end" means an end away from the tip of the heart. "Heart valve" and "prosthetic heart valve " have the same meaning.

As mentioned here, when the prosthetic heart valve is a mitral valve, " outflow tract" refers to a left ventricle outflow tract; when the prosthetic heart valve is a tricuspid valve, "outflow tract" refers to a right ventricle outflow tract. As mentioned here, "circle-like shape" refers to a hexagon and polygon with more than six edges and is close to a circle in nature, and "flower shape" refers to the shape of the outer edge of the petal. As mentioned here, the quadrate includes a rectangle as well as a square.

In the description of the present invention, it should be noted that orientations or position relationships indicated by terms " center" , "upper" , "lower" , "left" , "right" , "vertical" , "horizontal" , " inside " , " outside " and the like are orientations or position relationships shown in the drawings, and these terms are merely for facilitating description of the present invention and simplifying the description, but not for indicating or implying that the mentioned device or elements must have a specific orientation and must be established and operated in a specific orientation, and thus, these terms cannot be understood as a limitation to the present invention. Moreover, terms like "first", "second", "third" etc. are only used for description, not be considered as a designation or designation of relative importance.

In the description of the present invention, it should be noted that, unless otherwise clearly specified and limited, meanings of terms "install", "connected with", and "connected to" should be understood in a board sense. For example, the connection may be a fixed connection, a removable connection, or an integral connection; may be a mechanical connection or an electrical connection; may be a direct connection or an indirect connection by using an intermediate medium; or may be intercommunication between two components. For those of ordinary skill in the art, specific meanings of the above terms in the present invention may be understood based on specific situations.

In the description of the present invention, it should be noted that the term " or" is usually used to include the meaning of "and/or" , unless otherwise expressly stated.

The following further describes the present invention in combination with specific embodiments. It should be understood that these embodiments are only used for illustrating the present invention, but not for the limitation of the scope of the present invention. In practical application, the improvement and adjustment made by those skilled in the art according to the present invention still belong to the scope of protection of the present invention.

### Embodiment 1

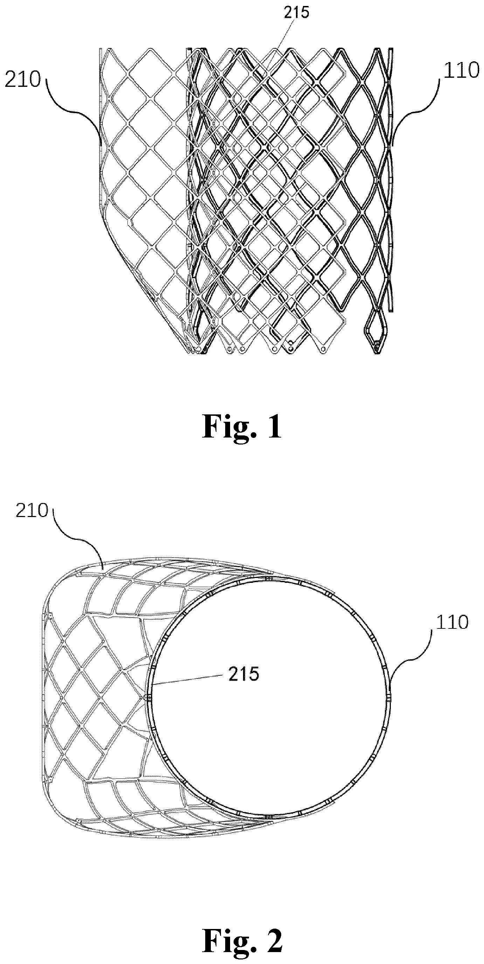

The present embodiment provides a prosthetic mitral valve. With reference to Figs. 1 to 12, a diagram of a heart valve of the present embodiment is shown, wherein the heart valve includes a stent body 110 for supporting prosthetic valve leaflets and an anchoring frame 210 for anchoring the stent body 110 to the annulus; the anchoring frame 210 is configured at a pre-set position located close to the left ventricle outflow tract, the stent body 110 is configured outside the anchoring frame 210 at a pre-set position located far away from the left ventricle outflow tract, and the anchoring frame 210 abuts against and is connected to the stent body 110.

The stent body 110 is configured inside with at least two prosthetic valve leaflets, wherein the number of the prosthetic valve leaflets may be the same as or different from the number of the native valve leaflets, and each of the valve leaflets is arranged circumferentially along the stent body 110. The valve leaflets may be fixed to the stent body 110 by way of direct connection or indirect connection, and free ends of the valve leaflet are engaged with each other. The material of the valve leaflets is the animal pericardium such as porcine pericardium or porcine pericardium or polymeric materials such as PET or PTFE. The number and the material of the prosthetic valve leaflets may be configured according to actual clinical needs, which will not be repeated here.

In the present embodiment, the stent body 110 and the anchoring frame 210 are of the left-right parallel connected structure, wherein compared with the existing single-layered stent, the stent body 110 and the anchoring frame 210 are used for supporting the valve leaflets and anchoring respectively, and a radial size of the stent body 110 is smaller so that the size of the prosthetic valve leaflet is also smaller, thereby improving the anti-fatigue performance of the valve leaflet. Since the size of the valve leaflet becomes smaller, the amount of the material of the valve leaflet is reduced. Compared with the traditional double-layered stent with the internal-external nested structure, the amount of the material of the anchoring frame 210 and the material of the skirt are less, so that the less the amount of the material is, the smaller the size is after pressing and holding, and the easier the stent is pressed, held and loaded. Studies have shown that if the prosthetic mitral valve may drive the blood to flow along a side wall of the ventricle, the blood may turn smoothly to create a large vortex, so as to eject the blood to the aorta and further to flow through the whole body. For the heart valve with the left-right structure, since the valve leaflets are located on the stent body and the stent body is biased more towards one side of the wall of the ventricle, the blood flow may tend to flow along the side wall of the ventricle, which is more beneficial to maintain a natural "blood vortex" for the left atrium, thereby promoting the recovery for the ventricle functions, especially for the vulnerable patients with severely-damaged heart conditions. In the present embodiment, a peripheral side of the anchoring frame 210 and a peripheral side of the stent body 110 abut against each other and are connected with each other at an abutting position 215. With reference to Figs. 1, 2, 3 and 6, Figs. 3 and 6 are illustrative perspective diagrams of the prosthetic heart valve respectively. The stent body 110 is a cylinder, and the anchoring frame 210 and the stent body 110 abut against each other from a distal edge to a proximal edge and are connected with each other at the abutting position 215. A surface-to-surface contact is formed between the anchoring frame 210 and the stent body 110, so the connection between the two are more firm. Naturally, in other embodiments, the stent body 110 and the anchoring frame 210 may also abut against and be connected with each other at a pre-set location between two ends. The abutting region and the abutting area between the stent body 110 and the anchoring frame 210, and the connecting sites between the stent body 110 and the anchoring frame 210, should be selected according to actual needs, which is not used to limit the protection scope of the present invention.

The anchoring frame 210 includes an inflow segment 211, a transition segment 212 and an outflow segment 213, wherein the inflow segment 211 is fixed in the left atrium, the transition segment 212 is fixed at the annulus and the outflow segment 213 extends into the left ventricle.

With continuous reference to Figs. 1, 2, 4, 6 and 7, in a preferred embodiment, at a side close to the left ventricle outflow tract, the outflow segment 213 is configured to extend in a direction far away from the left ventricle outflow tract. After the heart valve is implanted, the outflow segment 213 of the anchoring frame 210 is far away from the left ventricle outflow tract gradually, which may reduce the effects of the heart valve of the present embodiment on the functions of the heart, thereby preventing the conduction from being blocked.

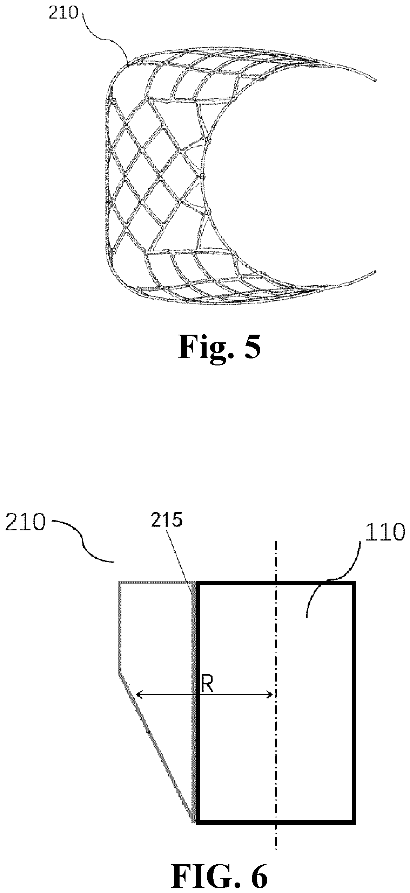

In the present embodiment, at the side close to the left ventricle outflow tract, the distance between the outflow segment 213 and the axis of the stent body 110 is R. From the distal end to the proximal end, R decreases gradually in a linear manner, as shown in Fig. 6, and then the outflow segment 213 is a slope. Naturally, in other embodiments, R may also optionally decrease gradually in a non-linear manner, e.g., being an arc, which may also reduce the risk in blocking the left ventricle outflow tract.

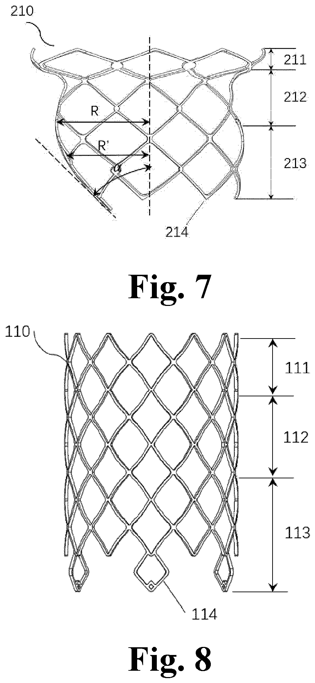

With continuous reference to Fig. 7, at the side close to the left ventricle outflow tract, an included angle between the tangent of the outflow segment 213 and the axial direction is α, α being an acute angle. If the included angle α is too small, the deviation of the outflow section 213 from the outflow tract will be too small, which increases the risk in the outflow tract obstruction; if the included angle α is too large, the resistance in pressing and holding will be increased, so the included angle α preferably ranges from 30° to 60°.

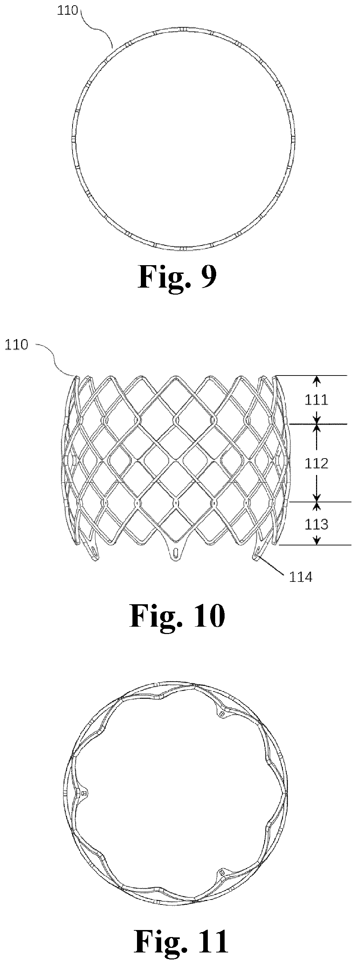

With reference to Fig. 8, in the present embodiment, the stent body 110 is a cylinder, including a main body inflow segment 111, a main body transition segment 112 and a main body outflow segment 113. Naturally, in other embodiments, the stent body 110 may also be an elliptical cylinder (as shown in Fig. 10), a cone, or a composite structure of the cylinder and the cone. In the present embodiment, a cross-section of the stent body 110 is a circle (as shown in Fig. 9); in other embodiments, the cross-section of the stent body 110 may also be configured to be a circle-like shape, a D-shape or a flower shape, or a composite structure. As shown in Fig. 11, the cross-section of the stent body 110 is the flower shape.

The stent body 110 is of a mesh structure constituted by a plurality of rows of units; in the present embodiment, the constituent unit is of a diamond structure, and the diamond mesh surface has the advantages of simple molding process and more smooth mesh surface. In other alternative embodiments, the constituent unit may also be a mesh unit that can be formed into an enclosed shape, such as a triangle, a quadrate, a pentagon, and a droplet shape. The stent body 110 is used to fix the valve leaflet, so the specific shape and the shape of the constituent unit may be selected according to the actual needs, and is not used to limit the scope of protection of the present invention.

With continuous reference to Figs. 8 and 10, the proximal end of the main body outflow segment 113 of the stent body 110 is further provided with a hanging tab 114, and the hanging tab 114 is used to be connected to a delivery system, so as to ensure that the relative position between the heart valve and the delivery system remains unchanged when the heart valve is loaded into the delivery system, the valve is released and removed from the delivery system and the valve is being transported. Naturally, in other embodiments, the hanging tab 114 may be further disposed at the distal end of the main body inflow segment 111, or at two ends of the stent body 110 simultaneously.

Since the heart valve is required to be implanted into the body via the delivery system, the stent body 110 should have a certain elastic deformation performance, and is pressed and held in the delivery system during transportation. The stent body 110 of the present embodiment is prepared by cutting a nickel-titanium alloy pipe, and the outer diameter of the pipe is 5mm to 15mm, wherein the shaped diameter size should be selected according to actual needs. In other embodiments, the material of the stent body 110 may further include an elastic deformable material or a plastic deformable material such as a balloon-expandable material, or may be a shape memory alloy that may respond to temperature changes to convert between a collapsed state and an expanded state. Specifically, the stent body 110 may adopt such as nitinol, titanium alloy, cobalt chromium alloy, MP35n, 316 stainless steel, L605, Phynox/Elgiloy and platinum chromium, or may be prepared by other bio-compatible metals known by those skilled in the art.

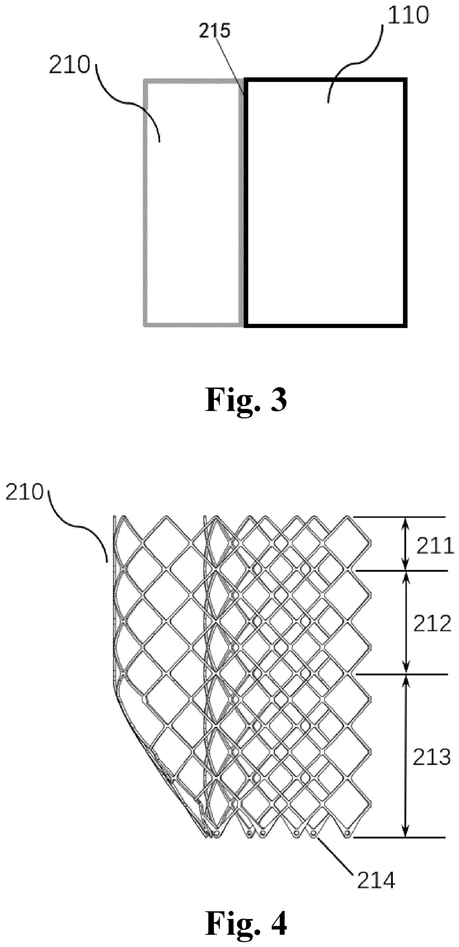

With continuous reference to Fig. 4, a structural schematic diagram of the anchoring frame 210 of the present embodiment is shown, wherein a peripheral side of the anchoring frame 210 may be pre-configured to have the same curvature as a peripheral side of the stent body 110, i.e., configured to be of a structure where a part of the peripheral side is sunken towards the center and then is fixed to the peripheral side of the stent body 1.

In an optional embodiment, the anchoring frame 210 is a cylinder, the anchoring frame 210 is connected to the peripheral side of the stent body 110, and after the connection is completed, the stent body 110 and the anchoring frame 210 form a structure where the two abut against each other. The anchoring frame 210 may be configured to be a cylinder, an elliptical cylinder, a cone, or a combination of the cylinder and the cone. A cross-section of the anchoring frame 210 may be a circle, a D-shape, a circle-like shape, a flower shape, or other irregular shapes, or a combination thereof.

The anchoring frame 210 is of a mesh structure constituted by a plurality of rows of units; in the present embodiment, the unit is a diamond unit. Naturally, in other embodiments, the unit may also be a mesh unit that can be formed into an enclosed shape such as a triangle, a quadrate, a pentagon, a circle-like shape and a droplet shape. The shape of the anchoring frame 210, and the shape, number and arrangement way of the unit should be selected according to actual needs, which is not limited here.

In another optional embodiment, a proximal end of the outflow segment 213 of the anchoring frame 210 is further provided with a fixing tab 214, and the fixing tab 214 is used to be connected to the delivery system, so as to ensure that the relative position between the heart valve and the delivery system remains unchanged when the heart valve is loaded into the delivery system, the valve is deployed and removed from the delivery system and the valve is being transported. Naturally, in other embodiments, the fixing tab 214 may be further disposed at a distal end of the inflow segment 211, or at two ends of the anchoring frame 210 simultaneously.

Since the heart valve is pressed and held into the delivery system for transportation, a stiffness of the anchoring frame 210 should be smaller than a stiffness of the stent body 110. Preferably, in the present embodiment, the anchoring frame 210 is prepared by weaving the nickel-titanium alloy, and the weaving process causes the anchoring frame 210 to have a better deformation performance for facilitating pressing, holding and transporting. Naturally, in other embodiments, the anchoring frame 210 may also adopt an elastic deformable material or a plastic deformable material such as a balloon-expandable material, or may be a shape memory alloy that may respond to temperature changes to convert between a collapsed state and an expanded state. For example, the anchoring frame may adopt nitinol, titanium alloy, cobalt chromium alloy, MP35n, 316 stainless steel, L605, Phynox/Elgiloy and platinum chromium, or may be prepared by other bio-compatible metals known by those skilled in the art.

In the present embodiment, the stent body 110 is connected to the anchoring frame 210 at the contact site by way of, specifically, riveting, welding, snapping and stitching, wherein suitable connection ways may be selected according to the materials of the stent body 110 and the anchoring frame 210.

Further, the heart valve of the present embodiment further includes a skirt for sealing. The skirt may be disposed on the inner surface of the stent body 110, or on the outer surface of the stent body 110, or on the inner surface and outer surface simultaneously; the skirt may be disposed or on the inner surface of the anchoring frame 210, or on the outer surface of the anchoring frame 210, or on the inner surface and outer surface simultaneously. The skirt is used for realizing sealing to ensure that a single channel of the blood is an outflow tract end flowing from an inflow tract end of the heart valve to the valve prosthesis, thereby effectively preventing the peripheral leakage and the regurgitation of the valve. The material of the skirt is the animal pericardium (such as porcine pericardium and bovine pericardium) or other bio-compatible polymeric materials (such as PET and PTFE). The region, the area and the material where the skirt is disposed should be configured according to actual clinical needs, which is not limited here.

In the present embodiment, the anchoring form for the valve prosthesis is not limited, and a flange may be disposed on the stent body 110 and the anchoring frame 210, wherein the anchoring form of Oversize is used; anchoring structures such as stabs and claws may also be disposed on the stent body 110 and the anchoring frame 210 for grabbing the primary tissue; the way in which the tether is anchored to the cardiac muscle wall is also used for fixing, or a combination of the anchoring ways may also be used for anchoring.

### Embodiment 2

The present embodiment provides a prosthetic tricuspid valve. In the present embodiment, the structure of the heart valve is similar to that of Embodiment 1, but differs from the latter in that the stent body 110 in the present embodiment is implanted at the annulus in the right ventricle inflow tract and the anchoring frame 210 is fixed at the pre-set position close to the right ventricle outflow tract.

In order to reduce the effects of the heart valve on the right ventricle outflow tract, the outflow segment 213 is configured to be far away from the right ventricle outflow tract gradually, thereby reducing the risk in blocking the right ventricle outflow tract.

### Embodiment 3

The present embodiment provides a prosthetic heart valve, which is an improvement made based on Embodiment 1 or Embodiment 2, wherein the abutting position 215 of the anchoring frame 210 and the stent body 110 is of the single-layered structure.

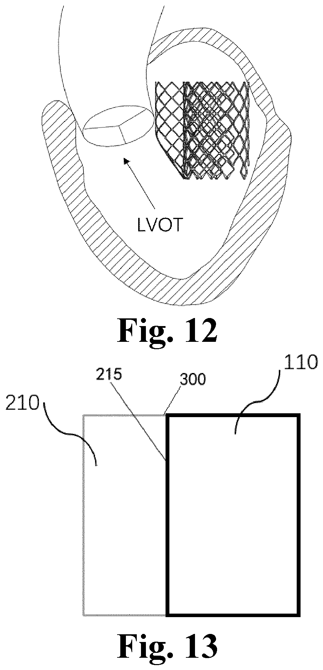

In an embodiment, with reference to Fig. 13, a diagram of the prosthetic heart valve of the present embodiment is shown, wherein the anchoring frame 210 is of a non-enclosed structure. In other words, the anchoring frame 210 is of an opening structure along an axial direction, with a cross-section of C-shape, wherein an opening end of the anchoring frame 210 in the axial direction is a frame opening end 300, and the frame opening end 300 abuts against and is connected to a periphery of the stent body 110.

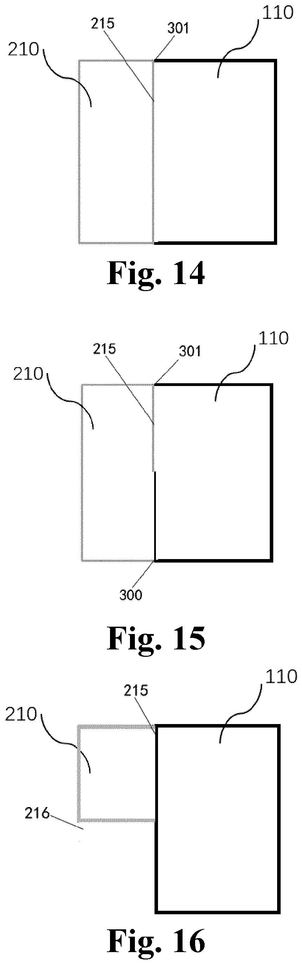

In another embodiment, with reference to Fig. 14, a diagram of the prosthetic heart valve of the present embodiment is shown, wherein the stent body 110 is of the non-enclosed structure, the stent body 110 is of an opening structure along the axial direction, with a cross-section of C-shape, wherein an opening end of the stent body 110 is a main body opening end 301, and the main body opening end 301 abuts against and is connected to a periphery of the anchoring frame 210.

In another optional embodiment, at the abutting position 215, a part of the anchoring frame 210 is of the non-enclosed structure while a part of the stent body 110 abutting against the part of the anchoring frame is of an enclosed structure, and other parts of the anchoring frame 210 are of enclosed structures while a part of the stent body 110 abutting against the other parts of the anchoring frame is of the non-enclosed structure. With reference to Fig. 15, an illustrative perspective diagram of the heart valve of the present embodiment is shown, the pre-set position of the proximal end of the anchoring frame 210 is opened and the pre-set position of the distal end of the stent body 110 is opened, wherein the frame opening end 300 abuts against the periphery of the stent body 110 and the main body opening end 301 abuts against the periphery of the anchoring frame 210, so that the abutting position 215 is of the single-layered structure.

The opening structure is disposed along the axial direction such that the amount of the materials for the valve stent and for the skirt covered on the valve stent is further reduced without affecting the functions of the anchoring frame 210 or the stent body 110, thereby further facilitating pressing, holding and releasing the valve prosthesis.

### Embodiment 4

The present embodiment provides a prosthetic heart valve, which is an improvement made based on Embodiment 1, Embodiment 2 or Embodiment 3, wherein the outflow segment of the anchoring frame 210 is configured with a notch structure 216, as shown in Fig. 16. The notch structure 216 is arranged towards a side of the outflow tract.

The notch structure 216 prevents the anchoring frame from crossing the ventricle wall surface, so that the prosthetic heart valve may not cause the ventricle wall to abut more close to a right side of the aortic valve or a left side of the pulmonary valve, thereby reducing the effects of the prosthetic heart valve on the functions of the heart.

The notch structure 216 reduces a subvalvular height of the outflow segment 213, thereby reducing the risk of the outflow tract obstruction. The shape and area of the notch structure 216 should be configured according to clinical needs, and are not used to limit the protection scope of the present invention.

The above disclosure is only the preferred embodiment of the present invention. The preferred embodiments do not describe all the details, and are not intended to limit the invention only to be the specific embodiments. It is obvious that various modifications and changes can be made to the content of the specification. The present invention selects and specifically describe the embodiments with the purpose of better explain the principle and practical use of the present invention, such that a person skilled in the art can well utilize the present invention. The present invention is merely limited by the appended claims and the scope and equivalents thereof.

## Claims

1. A prosthetic heart valve, comprising a stent body for supporting prosthetic valve leaflets and an anchoring frame for anchoring the stent body to annulus, wherein the anchoring frame is configured at a pre-set position located close to an outflow tract, the stent body is configured outside the anchoring frame and at a pre-set position located far away from the outflow tract, and the anchoring frame abuts against and is connected to the stent body.

2. The prosthetic heart valve according to claim 1, wherein a peripheral side of the anchoring frame and a peripheral side of the stent body abut against each other and are connected with each other at a position where the two abut against each other.

3. The prosthetic heart valve according to claim 1 or 2, wherein the position where the anchoring frame abuts against the stent body is of a single-layered structure.

4. The prosthetic heart valve according to claim 3, wherein the anchoring frame or the stent body is of a non-enclosed structure.

5. The prosthetic heart valve according to claim 4, wherein the anchoring frame is of an opening structure along an axial direction, and an opening end of the anchoring frame is connected to the stent body; or the stent body is of an opening structure along an axial direction, and an opening end of the stent body is connected to the anchoring frame.

6. The prosthetic heart valve according to claim 3, wherein at the position where the anchoring frame abuts against the stent body, a part of the anchoring frame is of an opening structure, a part of the stent body abutting against the part of the anchoring frame is of a non-opening structure, other parts of the anchoring frame are of non-opening structures, and a part of the stent body abutting against the other part of the anchoring frame is of the opening structure.

7. The prosthetic heart valve according to claim 1, 2, 4, 5 or 6, wherein the anchoring frame has a notch structure, the notch structure is located at an outflow segment of the anchoring frame, and the notch structure is configured to face towards the outflow tract.

8. The prosthetic heart valve according to claim 1, further comprising at least one connecting tab, wherein the heart valve is connected to a transporting system through the connecting tab; and the connecting tab is arranged at the stent body or at the anchoring frame.

9. The prosthetic heart valve according to claim 1, wherein the valve comprises prosthetic valve leaflets, and the valve leaflets are arranged at the main body portion.

10. The prosthetic heart valve according to claims 1, wherein the anchoring frame or the stent body is further provided with a skirt for sealing.
